(19) **Europäisches Patentamt European Patent Office Office européen des brevets**

(11) **EP 4 595 874 A1**

(12) **EUROPEAN PATENT APPLICATION**

(43) Date of publication:
06.08.2025 Bulletin 2025/32

(21) Application number: 24154736.3

(22) Date of filing: 30.01.2024

(51) International Patent Classification (IPC):
*A61B 5/00* (2006.01)    *A61B 5/02* (2006.01)
*A61B 5/1459* (2006.01)    *A61B 5/024* (2006.01)
*A61B 5/026* (2006.01)

(52) Cooperative Patent Classification (CPC):
A61B 5/1459; A61B 5/0075; A61B 5/02007;
A61B 5/0261; A61B 5/6852

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AG Eindhoven (NL)

(72) Inventors:
• LUCASSEN, Gerhardus Wilhelmus
  Eindhoven (NL)
• MUELLER, Manfred
  Eindhoven (NL)
• BABIC, Drazenko
  5656AG Eindhoven (NL)

(74) Representative: Philips Intellectual Property &
Standards
High Tech Campus 52
5656 AG Eindhoven (NL)

(54) **INTRAVACULAR MEASUREMENT OF BLOOD OXYGEN SATURATION**

(57)    A system (100) for determining blood oxygen saturation, is provided. The system comprises one or more processors (110) configured to: receive optical data (120) representing an optical spectrum of an intravascular region (130) over one or more wavelength intervals; analyze the optical data (120) to determine a measurement of blood oxygen saturation (140); and output the measurement of blood oxygen saturation (140).

FIG. 1

EP 4 595 874 A1

**Description**

TECHNICAL FIELD

The present disclosure relates to determining an intravascular measurement of blood oxygen saturation. A system, a computer-implemented method, and a computer program product, are disclosed.

BACKGROUND

Ischemic stroke is a leading cause of disability worldwide. Ischemic stroke is caused by cerebral ischemia, i.e. the deprivation of blood flow to the brain. The principal cause of cerebral ischemia is thromboembolism. Ischemic stroke occurs when a thrombus, also known as a blood clot, develops outside of the brain and is carried via the vasculature to the brain where it forms a blockage. The blockage, also known as an occlusion, leads to cerebral ischemia, and hence to ischemic stroke. Another cause of cerebral ischemia is atherosclerosis; a process in which fatty deposits accumulate on the inner walls of the arteries. The fatty deposits are known as atheromas, or plaque. Plaque can grow within a cerebral artery to form a plaque-type occlusion that deprives the brain of blood, similarly leading to cerebral ischemia, and hence to ischemic stroke. This type of atherosclerosis is referred-to as intracranial atherosclerosis. Intracranial atherosclerosis is rare in Western populations but more prevalent in Asian, Black and Hispanic populations.

Within a period of minutes to hours, an occlusion within a cerebral artery typically results in a severely ischemic "core" region of brain tissue that is irreversibly damaged. The core is surrounded by a less-ischemic "penumbra" of surviving brain tissue that incurs a risk of infarction, i.e. a risk of tissue death as a result of insufficient blood supply. This condition is known as ischemic stroke. Sometimes the onset of infarction is delayed by blood flow to the ischemic tissue via a subsidiary network of vascular channels referred to as the cerebral collateral circulation. However, this response takes time to become effective, and so parts of the penumbra may become infarcted during the period following ischemic stroke.

Fortunately, if the occluded artery is treated in a timely manner, the penumbra can be "reperfused" and the resulting ischemic lesion can remain small or absent.

There are two factors that help in preserving the blood flow distally to the clot location: capacity of the collateral circulation and the clot permeability.

The current treatment options for cerebral ischemia include the use of a clot-busting medicine to disburse a blood clot-type occlusion; stenting; and mechanical thrombectomy, i.e. an interventional procedure in which an occlusion is mechanically removed from the body. A variety of mechanical thrombectomy devices are available, including aspiration catheters that are used to suck an occlusion from the vasculature, stent retrievers that include an expandable stent which is used to capture and subsequently extract an occlusion from the body, and expandable mesh disks. Some of these treatment options are better suited to some types of occlusions than others. For instance, stenting is typically used to treat plaque-type occlusions, whereas mechanical thrombectomy is typically used to blood clot-type occlusions. It is possible to further optimize the choice of treatment based on the type of tissue present in a blood clot-type occlusion. For example an aspiration catheter is typically better-suited to removing red blood cell-poor blood clots, whereas most stent retrievers are typically better-suited to removing red blood-cell-rich blood clots.

The most important factor in treating cerebral ischemia is early treatment. In order to carry-out early treatment, a physician requires information relating to the occlusion. For instance, having information relating to the amount of perfusion in tissue surrounding an occlusion would enable the physician to assess brain tissue viability around the location of the occlusion. Having information relating to the type of tissue present in the occlusion would enable the physician to make a first-time-right choice of the various treatment options described above and thereby perform early treatment.

Conventionally, the various treatment options for cerebral ischemia have been evaluated using non-invasive imaging techniques such as projection X-ray imaging, computed tomography "CT" imaging, and magnetic resonance imaging "MRI". For instance, digital subtraction angiography, "DSA" is the current gold standard for assessing the cerebral collateral circulation. However, these techniques have limitations. For instance, DSA images only depict vessels that include contrast agent. This makes it hard to even determine the length of an occlusion because only the proximal edge of an occlusion is visible in DSA images. Moreover, these imaging techniques typically provide little or no information on the type of tissue present in an occlusion.

Thus, there remains a need to provide information relating to vascular occlusions in order to support the evaluation of options for their treatment.

SUMMARY

According to one aspect of the present disclosure, a system for determining blood oxygen saturation, is provided. The system includes one or more processors configured to:

receive optical data representing an optical spectrum of an intravascular region over one or more wavelength intervals;

analyze the optical data to determine a measurement of blood oxygen saturation; and

output the measurement of blood oxygen saturation.

The above-described system provides a measurement of blood oxygen saturation in an intravascular region. The measurement of blood oxygen saturation is a useful parameter in assessing the level of perfusion to tissue surrounding the intravascular region. The system may be used to evaluate the level of perfusion to tissue surrounding an occlusion, for example. The system therefore supports the physician in making a first-time-right choice of the various treatment options for treating the occlusion.

Further aspects, features, and advantages of the present disclosure will become apparent from the following description of examples, which is made with reference to the accompanying drawings.

BRIEF DESCRIPTION OF THE DRAWINGS

Fig. 1 is a schematic diagram illustrating an example of a system 100 for determining blood oxygen saturation, in accordance with some aspects of the present disclosure.

Fig. 2 is a flowchart illustrating an example of a computer-implemented method of determining blood oxygen saturation, in accordance with some aspects of the present disclosure.

Fig. 3 is an example of the optical signatures of various chromophores that are typically present in the vasculature, in accordance with some aspects of the present disclosure.

Fig. 4 is an example of an outputted measurement of blood oxygen saturation 140, in accordance with some aspects of the present disclosure.

Fig. 5 is a schematic diagram illustrating an example of a proximal position 150, and a distal position 180, with respect to an occlusion 160 in a blood vessel 170, in accordance with some aspects of the present disclosure.

Fig. 6 is a schematic diagram illustrating an example of an intravascular optical probe 240 disposed in a distal position 180 with respect to an occlusion 160 in a blood vessel 170, in accordance with some aspects of the present disclosure.

Fig. 7 is an example of a graphical representation 250 of a blood vessel 170 including an indication of intravascular optical probe positions $210_{1...i}$ and corresponding measurements of blood oxygen saturation, in accordance with some aspects of the present disclosure.

Fig. 8 is an example of the optical signatures of various types of blood clot, blood, a vessel wall, and plaque, in accordance with some aspects of the present disclosure.

Fig. 9 is a schematic diagram illustrating an example of an outputted indication of various types of tissue 190, in accordance with some aspects of the present disclosure.

Fig. 10 is a schematic diagram illustrating an example of an outputted indication of a measurement of blood oxygen saturation 140 and an outputted indication of a red blood cell fraction, of an intravascular region, in accordance with some aspects of the present disclosure.

Fig. 11 is a schematic diagram illustrating a first example of an intravascular optical probe 240 for insertion into the vasculature, in accordance with some aspects of the present disclosure.

Fig. 12 is a schematic diagram illustrating a second example of an intravascular optical probe 240 for insertion into the vasculature, in accordance with some aspects of the present disclosure.

DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system in which optical data representing an optical spectrum of an intravascular region is analyzed in order to determine a measurement of blood oxygen saturation within a cerebral artery. The measurement of blood oxygen saturation is obtained for the purpose of supporting a physician in evaluating the treatment options for ischemic stroke. However, it is to be appreciated that unless explicitly stated, the use of the system disclosed herein is not limited to these examples. Thus, the system may be used to determine measurements of blood oxygen saturation within blood vessels in the anatomy in general, and the system may be used in various clinical applications. For instance, the system may be used to determine measurements of blood oxygen saturation within blood vessels such as arteries, or veins, and such blood vessels may be in anatomical regions such as the brain, the neck, the arm, the leg, and so forth. Moreover, the system may be used in clinical applications such as the evaluation of treatment options for peripheral vascular disease, and so forth, as well as ischemic stroke.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

It is also noted that some operations that are described as being performed by the one or more processors of the systems disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there remains a need to provide information relating to vascular occlusions in order to support the evaluation of options for their treatment.

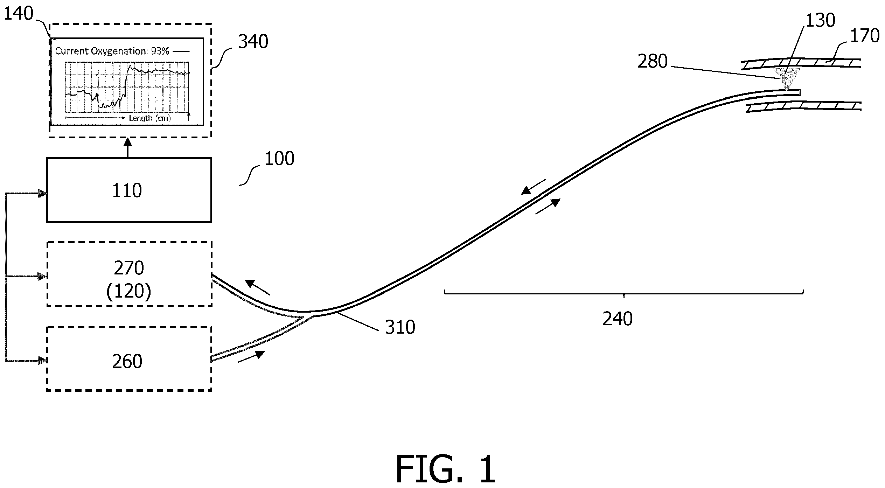

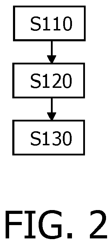

Fig. 1 is a schematic diagram illustrating an example of a system 100 for determining blood oxygen saturation, in accordance with some aspects of the present disclosure. Fig. 2 is a flowchart illustrating an example of a computer-implemented method of determining blood oxygen saturation, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for determining blood oxygen saturation comprises one or more processors 110 configured to:

receive S 110 optical data 120 representing an optical spectrum of an intravascular region 130 over one or more wavelength intervals;
analyze S120 the optical data 120 to determine a measurement of blood oxygen saturation 140; and
output S130 the measurement of blood oxygen saturation 140.

The above-described system provides a measurement of blood oxygen saturation in an intravascular region. The measurement of blood oxygen saturation is a useful parameter in assessing the level of perfusion to tissue surrounding the intravascular region. The system may be used to evaluate the level of perfusion to tissue surrounding an occlusion, for example. The system therefore supports the physician in making a first-time-right choice of the various treatment options for treating the occlusion.

The operations that are performed by the one or more processors 110 of the system 100 are described in more detail below.

With reference to the method illustrated in Fig. 2, in the operation S110, the one or more processors 110 of the system 100 receive optical data 120. The optical data 120 represents an optical spectrum of an intravascular region 130 over one or more wavelength intervals.

In general, the optical data 120 that is received in the operation S 110 may represent an optical property such as the reflectance, optical scatter, transmission, or absorption, of the intravascular region 130. In general, the optical data 120 may be generated in response to the irradiation of the intravascular region with optical radiation. In general, the one or more wavelength intervals may occupy a portion of the visible region of the optical spectrum and/or a portion of the near-infrared region of the optical spectrum. In general, the optical data 120 may be generated in real-time, i.e. the optical data 120 may be real-time optical data. Alternatively, the optical data 120 may have been generated historically, i.e. the optical data 120 may be historic optical data.

The optical data 120 that is received in the operation S110 may be received from various sources. For example the optical data 120 may be received from a computer readable storage medium, or from the Internet, or the Cloud, and so forth. In the system 100 illustrated in Fig. 1, the optical data 120 is received from an optical detector 270 and the optical data is generated in real-time. In general, the optical data 120 that is received in the operation S110 may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

With continued reference to the method illustrated in Fig. 2, in the operation S120, the one or more processors 110 of the system 100, analyze S120 the optical data 120 to determine a measurement of blood oxygen saturation 140.

Blood oxygen saturation is a measurement of the amount of oxygen that is carried by the blood. Blood oxygen saturation is typically defined as the fraction of oxygen-saturated haemoglobin relative to total (saturated plus unsaturated) haemoglobin in the blood. Blood oxygen saturation may be measured in arterial blood, in venous blood, and in entire blood in tissue, and is denoted $SaO_2$, $SvO_2$, and $StO_2$, respectively. Arterial blood oxygen saturation, $SaO_2$, is defined by Equation 1:

$$SaO_2 = \frac{HbO_2}{HbO_2 + Hb} \qquad\qquad\qquad \text{Equation 1}$$

wherein $HbO_2$ represents the amount of oxygen-saturated haemoglobin, and $Hb$ represents the amount of unsaturated, or deoxygenated, haemoglobin in the tissue. Values for blood oxygenation in venous blood, and in entire blood in tissue, may also be obtained using Equation 1 using corresponding measurements obtained from venous blood, and entire blood in tissue, respectively.

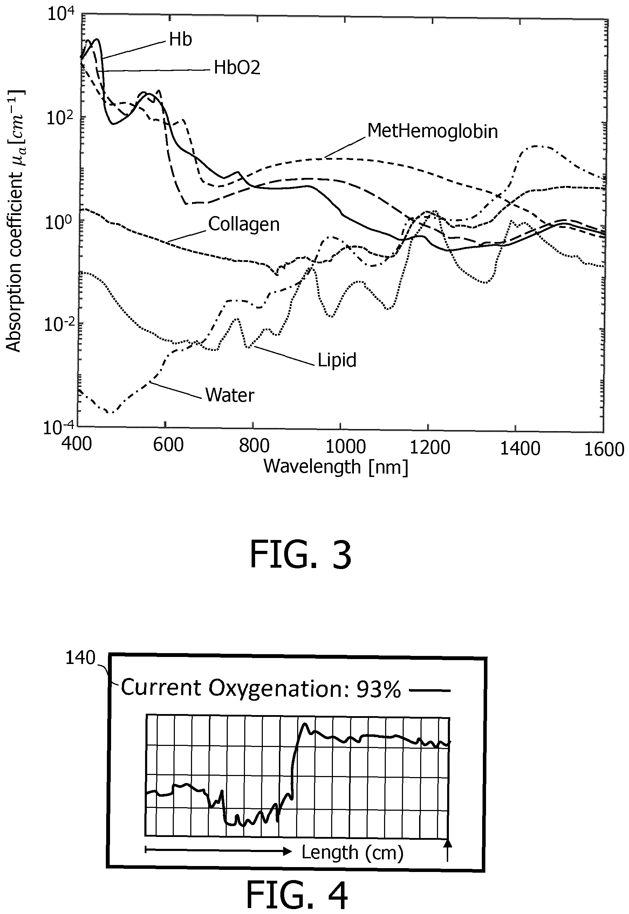

Measurements of blood oxygen saturation may be obtained by analyzing an optical spectrum of blood, and determining the contributions to the optical spectrum from oxygen-saturated haemoglobin, i.e. $HbO_2$, and from unsaturated, or deoxygenated, haemoglobin, i.e. $Hb$. An example of this analysis is now described with reference to Fig. 3, which is an example of the optical signatures of various chromophores that are typically present in the vasculature, in accordance with some aspects of the present disclosure.

The abscissa in Fig. 3 represents wavelength in nanometers, and the ordinate axis of Fig. 3 represents the absorption coefficient of the various chromophores. The chromophores represented in Fig. 3 include water, lipid, collagen and various haemoglobin chromophores. Haemoglobin is present in the vasculature in various forms, including as oxygenized haemoglobin, labelled $HbO_2$ in Fig. 3, deoxygenized haemoglobin, labelled $Hb$ in Fig. 3, and as methaemoglobin. As may be seen in Fig. 3; within a range extending from approximately 450 nanometers to approximately 800 nanometers, the absorption coefficients of oxygenized haemoglobin, $HbO_2$, and deoxygenized haemoglobin, $Hb$, are relatively high compared to those of other chromophores that are present in the vasculature. In this context, the term "approximately" refers to within $\pm$ 10 nanometers, or within $\pm$ 5 nanometers, of these values. As may also be seen in Fig. 3; there are also various absorption peaks that are within the range extending from approximately 450 nanometers to approximately 800 nanometers that are narrower than this range, and which are characteristic of oxygenized haemoglobin, i.e. $HbO_2$, and deoxygenized haemoglobin, i.e. $Hb$. For instance, deoxygenized haemoglobin, $Hb$, has a distinctive absorption peak extending from approximately 700 nanometers to approximately 800 nanometers, and oxygenized haemoglobin, $HbO_2$, has a distinctive absorption peak extending from approximately 600 nanometers to approximately 700 nanometers. Thus, a measurement of the blood oxygen saturation of an intravascular region may be determined based on a contribution to the optical absorption coefficient caused by oxygenized haemoglobin, $HbO_2$, and by deoxygenized haemoglobin, $Hb$, over one or more wavelength intervals (such as over the wavelength interval extending from approximately 700 nanometers to approximately 800 nanometers, and such as over the wavelength interval extending from approximately 600 nanometers to approximately 700 nanometers) that are within, i.e. less than or equal to, a range extending from approximately 450 nanometers to approximately 800 nanometers. As may be appreciated from Fig. 3, there are also other wavelength intervals than these examples, that are likewise within a range extending from approximately 450 nanometers to approximately 800 nanometers, and over which the optical absorption may be measured to determine the blood oxygen saturation.

As may be appreciated, instead of measuring the optical absorption as illustrated in Fig. 3, measurements of other optical properties of the intravascular region 130 may alternatively be used to determine the measurement of blood oxygen saturation 140. For instance, measurements of optical properties such as reflectance, or optical scattering, or transmission, of the intravascular region, are also affected by the chromophores represented in Fig. 3. Consequently measurements of these optical properties may alternatively be used to determine a measurement of blood oxygen saturation 140. The measurements of such optical properties may be obtained using the intravascular tissue analysis system 100 illustrated in Fig. 1, as described in more detail below.

Various techniques may be used to analyze the optical data 120 in the operation S120 in order to determine the measurement of blood oxygen saturation 140. In one example, a model fitting technique is used to fit an optical model to an optical spectrum of the intravascular region 130. In this example, the operation of analyzing S120 the optical data 120 comprises: fitting an optical model to the optical spectrum; extracting one or more model parameters from the fitted optical model; and determining the measurement of blood oxygen saturation 140 based on the one or more extracted model parameters.

An example of an optical model that may be used in this example is the optical model reported in a document by Farrel, T. J. et al., "A diffusion theory model of spatially resolved, steady-state diffuse reflectance for the noninvasive determination of tissue optical properties in vivo", Medical Physics 19(4): p. 879-888 (1992).

The Farrell model is derived from diffusion theory and describes the optical properties of the tissue via two properties, the absorption coefficient $\mu_a(\lambda)$ and the reduced scattering coefficient $\mu'_s(\lambda)$, both of which are dependent on the wavelength $\lambda$, of the optical radiation. The total absorption coefficient $\mu_a^{total}(\lambda)$ is given by the sum of the absorption coefficients of the various absorbers weighted by their concentrations c, i.e.:

$$\mu_a^{total}(\lambda) = c_{Haemoglobin}\mu_a^{Haemoglobin}(\lambda) + c_{Lipid}\mu_a^{Lipid}(\lambda) + c_{Water}\mu_a^{Water}(\lambda)$$
$$+ \cdots. \qquad \text{(Equation 1)}$$

The wavelength-dependent absorption coefficients of Lipids $\mu_a^{Lipid}(\lambda)$, Water $\mu_a^{Water}(\lambda)$, and other absorbers are known from literature. The absorption spectrum of deoxygenated haemoglobin, Hb, is different to that of oxygenated haemoglobin, $HbO_2$. The absorption coefficient due to haemoglobin, $\mu_a^{Haemoglobin}(\lambda)$ is given by:

$$c_{Haemoglobin}\mu_a^{Haemoglobin}(\lambda)$$
$$= \nu(\lambda)\left[StO_2 c_{Hbo2}\mu_a^{HbO_2}(\lambda) + (1 - StO_2)c_{Hb}\mu_a^{Hb}(\lambda)\right] \qquad \text{(Equation 2)}$$

where $\mu_a^{Hb}(\lambda)$ and $\mu_a^{HbO_2}(\lambda)$ correspond to absorption coefficients of deoxygenized and oxygenized haemoglobin, and $StO_2$ is the oxygen saturation level of the tissue. The parameter $\nu(\lambda)$ is used to account for the inhomogeneous distribution of haemoglobin and is known as the "pigment packaging factor" and is given by:

$$\nu(\lambda) = \frac{1 - \exp\left(-2R\left[StO_2\mu_a^{HbO_2}(\lambda) + (1 - StO_2)\mu_a^{Hb}(\lambda)\right]\right)}{2R\left[StO_2\mu_a^{HbO_2}(\lambda) + (1 - StO_2)\mu_a^{Hb}(\lambda)\right]} \qquad \text{(Equation 3)}$$

as reported in a document by Verkruysse, W., et al., "Modelling light distributions of homogeneous versus discrete absorbers in light irradiated turbid media", Phys. Med. Biol. 42, 51, and wherein R corresponds to the average radius of a haemoglobin concentration, e.g., a blood vessel. The reduced scattering coefficient is empirically modeled as the sum of Mie and Rayleigh scattering:

$$\mu'_s(\lambda) = \alpha\left[\rho\left(\frac{\lambda}{\lambda_0}\right)^{-b} + (1 - \rho)\left(\frac{\lambda}{\lambda_0}\right)^{-4}\right] \qquad \text{(Equation 4)}$$

where $\lambda_0$ = 800 nm corresponds to a wavelength normalization value, $\alpha$ is the reduced scattering amplitude at $\lambda_0$ the Mie scattering slope is b, and $\rho$ denotes the Mie-fraction of the reduced scattering in the tissue.

The Farrel model may be fitted to the absorption spectra using a Levenberg-Marquardt non-linear inversion algorithm, for example. The result of the fitting is to determine the values of the concentrations of the haemoglobin chromophores, i.e. $c_{Hb}$ and $c_{HbO2}$, which represent the contributions to the optical absorption coefficient caused by oxygenized haemoglobin, $HbO_2$, and by deoxygenized haemoglobin, $Hb$, respectively.

Instead of using the optical model described in the above example, other optical models, and also other fitting techniques, may alternatively be used in the operation S120 to analyze the optical data 120 in order to determine the measurement of blood oxygen saturation 140. For instance, in one example, a pre-determined partial least squares "PLS" regression vector, RV, is used to extract the model parameters. In this example, the model parameters $Y_{pred}$ for a newly-acquired optical spectrum are calculated by multiplying the newly-acquired optical spectrum of an intravascular region 130 spectrum $X_{new}$ by the regression vector; i.e. $Y_{pred} = X_{new}RV$. The pre-determined partial least squares regression vector RV may be obtained using measured, or synthetic, spectra of media within the intravascular region 130, such as those illustrated in Fig. 3, and performing a PLS regression analysis. In another example, a machine learning algorithm such as a neural network, a decision tree, a support vector machine "SVM", principal component analysis, and so forth, may be used to analyze the optical data 120. The training data used to train such machine learning algorithms may include multiple sets of optical spectra of intravascular regions, and for each set of optical spectra, corresponding ground truth data comprising a ground truth value for the oxygenized haemoglobin, $HbO_2$, and deoxygenized haemoglobin, $Hb$, content, or alternatively a ground truth value for the measurement of blood oxygen saturation 140. The ground truth data, may be determined by blood gas analysis, or (finger) pulse oximetry.

Returning to the method illustrated in Fig. 2, in the operation S 130, the one or more processors 110 of the intravascular tissue analysis system 100 output the measurement of blood oxygen saturation 140. The measurement of blood oxygen saturation 140 may be outputted in various forms in the operation S130. For example, the red blood cell content 140 and the lipid content 150 may be outputted in numerical form, or in textual form, or in the form of an icon, or in the form of a graph. The measurement of blood oxygen saturation 140 may be outputted in real-time in the operation S130.

By way of an example, Fig. 4 is an example of an outputted measurement of blood oxygen saturation 140, in accordance with some aspects of the present disclosure. In the upper portion of Fig. 4, the blood oxygen saturation 140 is outputted as numerical value for an intravascular region 130 that corresponds to a current position of an intravascular optical probe in vessel.

The measurement of blood oxygen saturation 140 may be outputted to various media in the operation S 130, including to a display such as to the display 340 illustrated in Fig. 1, or to a virtual/ augmented reality display device, or to a printer, or to a computer-readable storage medium, or to the Internet, or to the Cloud, and so forth.

In summary, the above-described system provides a measurement of blood oxygen saturation in an intravascular region. The measurement of blood oxygen saturation is a useful parameter in assessing the level of perfusion to tissue surrounding the intravascular region. The system may be used to evaluate the level of perfusion to tissue surrounding an occlusion, for example. The system therefore supports the physician in making a first-time-right choice of the various treatment options for treating the occlusion.

Various further examples of the system 100 are described below.

In one example, the one or more processors 110 are configured to:

determine, based on the measurement of blood oxygen saturation 140, a perfusion status and/or a blood flow status of the intravascular region 130; and
output an indication of the perfusion status and/or the blood flow status, respectively.

An example of the perfusion, and blood flow, status that may be outputted in accordance with this example is the outputting of indications such as "total occlusion", or "leaking blood clot" or "functioning collaterals". Such indications may be generated by applying one or more thresholds to the measurements of blood oxygen saturation 140. For instance, the indication "total occlusion", may be outputted if the measured value of blood oxygen saturation 140 is less than 30%. The indication "leaking blood clot" may be outputted if the measured value of blood oxygen saturation 140 is between 30% and 60%. The indication "functioning collaterals" may be outputted if the measured value of blood oxygen saturation 140 is above 60% .

The outputted perfusion status, and blood flow status, support the physician in making a first-time-right choice of the various treatment options for treating the occlusion.

In another example, the one or more processors 110 are configured to:

classify, based on the measurement of blood oxygen saturation 140, the intravascular region 130 as representing a proximal position 150 with respect to an occlusion 160 in a blood vessel 170, or as a distal position 180 with respect to an occlusion 160 in a blood vessel 170; and

output an indication of the classification.

In this example, the classification between a proximal position and a distal position may be determined by applying a threshold to the measurement of blood oxygen saturation 140. Relatively lower values of blood oxygen saturation 140 are indicative of distal positions, whereas relatively higher values of blood oxygen saturation 140 are indicative of proximal positions. Immediately adjacent to an occlusion, the blood oxygen saturation may be relatively lower in the distal position. In this situation, a history of the measurements may be used to classify a position as a proximal position, or a distal position. For instance if the blood oxygen saturation 140 measurements decrease whilst the intravascular optical probe is pulled back, i.e. in a direction against anterograde blood flow, an intravascular region 130 may be deemed to represent a distal position with respect to an occlusion in a blood vessel. On the other hand, if the blood oxygen saturation 140 measurements increase whilst the intravascular optical probe is pulled back, an intravascular region 130 may be deemed to represent a proximal position with respect to an occlusion in a blood vessel.

In another example, the optical data 120 that is received in the operation S110 represents an optical spectrum of the intravascular region 130 at each of a plurality of points in time. In this example, various analyses are performed on the optical data. In one variant of this example, the analyzing comprises:

applying a temporal filter to at least a portion of the optical spectrum to provide one or more corresponding temporally-filtered spectra; and
determining the measurement of blood oxygen saturation 140 using the temporally-filtered spectra.

In this variant, temporal filtering is used to provide a smoothed measurement of blood oxygen saturation 140. The complete optical spectrum may be filtered, or alternatively, only a portion of the optical spectrum may be filtered. For instance, the portion of the spectrum which is subject to temporal variation may be filtered. This variant may be used to reduce the impact of interfering effects such as the beating heart, interference, and noise, on the measurements. An example of a temporal filter that may be used in this example is the calculation of the measurement of blood oxygen saturation 140 as a moving average.

In another variant of this example, the analyzing comprises:

applying a temporal filter to the measurements of blood oxygen saturation 140, to provide temporally filtered measurements of blood oxygen saturation 140; and
wherein the outputting the measurement of blood oxygen saturation 140 comprises outputting the temporally filtered measurements of blood oxygen saturation 140.

In this variant, temporal filtering is likewise used to provide a smoothed measurement of blood oxygen saturation 140. However, in contrast to the variant immediately above, the smoothing is applied to the measurements, rather than to the optical data.

In another variant of this example, the analyzing comprises:
extracting a pulsatile signal from temporal variations in the optical spectrum, and outputting the pulsatile signal.

In this variant, the pulsatile signal may represent the beating of the heart. Thus, this variant, may be used to provide a cardiac signal. The cardiac signal may be used to determine the heart rate of a subject, for example. The cardiac signal may also be used provide information on the nature of the blood flow in the intravascular region. This information therefore supports the physician in making a first-time-right choice of the various treatment options for treating the intravascular region.

In another variant of this example, the analyzing comprises:
extracting a pulsatile signal from temporal variations in the measurement of blood oxygen saturation 140, and outputting the pulsatile signal.

In this variant, the pulsatile signal may likewise represent the beating of the heart. However, in contrast to the variant immediately above, the pulsatile signal is extracted from the measurements of blood oxygen saturation 140, rather than from the optical data.

In another example, the blood oxygen saturation 140 is outputted for each of multiple positions along a vessel. In this example, the optical data 120 represents an optical spectrum of the intravascular region at each of a plurality of positions $210_{1...i}$ along a blood vessel. In this example, the one or more processors 110 are configured to perform the analyzing, and the outputting, for a plurality of the positions along the blood vessel.

An example of the outputting of measurements of blood oxygen saturation in accordance with this example is illustrated by the graph in the lower portion of Fig. 4. In this graph, the abscissa represents the position along the vessel, and the ordinate represents the measurement of blood oxygen saturation 140. The optical data 120 in this example may be obtained during a so-called pullback procedure in which an intravascular optical probe 240 that is used to obtain the optical data 120 is translated along the vessel. The position along the vessel may be determined based on a known pullback speed of the optical probe.

In a related example, the one or more processors 110 are configured to evaluate a comparison of the measurements of blood oxygen saturation 140 between two or more of the positions. The comparison that is evaluated in this example may be a difference, or a ratio, between the measurements, for example.

In another related example, the plurality of positions comprises a proximal position 150 with respect to an occlusion 160 in the blood vessel 170, and a distal position 180 with respect to the occlusion 160 in the blood vessel 170.

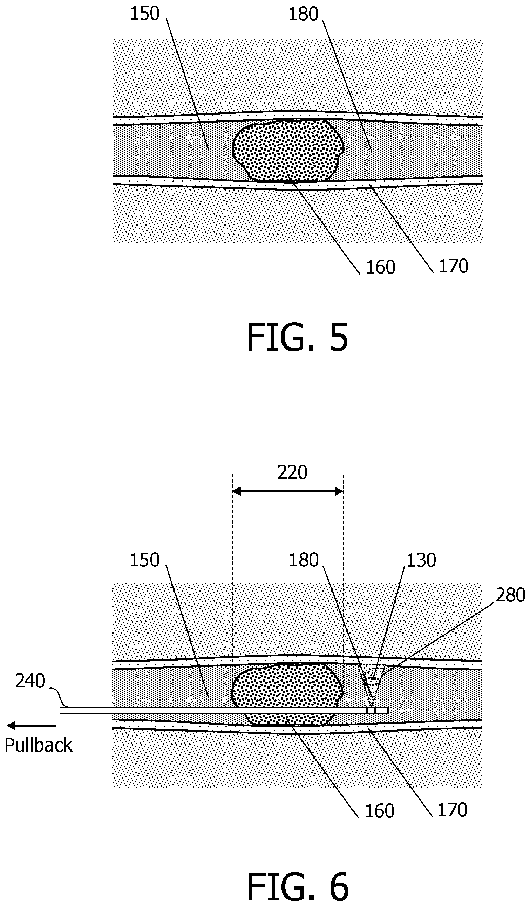

In this example, the system may be used to evaluate a comparison of the measurements of blood oxygen saturation 140 between a proximal position 150 with respect to an occlusion 160 in a blood vessel 170, and a distal position 180 with respect to the occlusion 160 in the blood vessel 170. This example is described with reference to Fig. 5, which is a schematic diagram illustrating an example of a proximal position 150, and a distal position 180, with respect to an occlusion 160 in a blood vessel 170, in accordance with some aspects of the present disclosure. The occlusion 160 illustrated in Fig. 5 may for example be a blood clot-type occlusion, or a plaque-type occlusion, as described above. The blood vessel may 170 be an intracranial artery, for example. The blood vessel may be surrounded by cerebral tissue. In this context, the terms proximal, and distal, respectively refer to positions that are relatively upstream, and relatively downstream, with respect to the direction of conventional blood flow in the vessel.

In this example, the measurements of blood oxygen saturation 140 in the proximal and distal positions provide valuable information to a physician relating to perfusion of tissue surrounding the occlusion. For instance, in some cases, an occlusion is total and there is negligible blood flow in proximal and distal positions that abut the occlusion. In such positions, the measurements of blood oxygen saturation are therefore both negligible. In proximal and distal positions that are relatively further away from a total occlusion 160, the measurements of blood oxygen saturation may both be relatively higher, the latter due to cerebral collateral circulation. The comparison of the measurements of blood oxygen saturation 140 between proximal and distal positions with respect to a total occlusion therefore quantifies the amount of collateral circulation around the total occlusion. In other cases, the occlusion may be partial rather than total. In such cases, relatively higher measurements of blood oxygen saturation may be observed both proximally and distally with respect to the partial occlusion. Thus, the comparison of the measurements of blood oxygen saturation 140 between proximal and distal positions with respect to a partial occlusion quantifies the amount of flow through the partial occlusion.

The measurements of the blood oxygen saturation 140 in the proximal and distal positions may be obtained using an intravascular optical probe such as the intravascular optical probe 240 illustrated in Fig. 6. Fig. 6 is a schematic diagram illustrating an example of an intravascular optical probe 240 disposed in a distal position 180 with respect to an occlusion 160 in a blood vessel 170, in accordance with some aspects of the present disclosure. The intravascular optical probe 240 illustrated in Fig. 6 is described in more detail below. In short, the intravascular optical probe 240 acquires optical data 120 representing an optical spectrum of an intravascular region 130 by irradiating the intravascular region 130 with optical radiation 280. The optical data 120 may be obtained during a so-called pullback procedure in which the optical probe 240 is withdrawn from the vasculature, as illustrated by the arrow labelled Pullback. The optical data 120 is then analyzed using the technique described above to determine the blood oxygen saturation 140.

In a related example, a value of a length 220 of an occlusion and/or a measure of ischemia 230 resulting from an occlusion, are estimated. In this example, the one or more processors 110 are configured to:

> estimate, based on the measurements of blood oxygen saturation 140 at the plurality of positions along the blood vessel, a length 220 of an occlusion and/or a measure of ischemia 230 resulting from an occlusion; and
> output the value of the length of the occlusion and/or the measure of ischemia.

In positions that are close to the occlusion 160, a relatively lower value of the blood oxygen saturation 140 is expected, whereas further away from the occlusion 160, a relatively higher value is expected. The estimate of the length of the occlusion may therefore be determined by calculating, based on the pullback speed of the optical probe 240 used in the pullback procedure, a length of the vessel over which the measurements of blood oxygen saturation 140 are below a threshold level. The length of an occlusion is a useful parameter in assessing an occlusion. For instance, the occlusion length may be used to determine a size of a stent to use to treat the vessel after the occlusion has been removed.

An example of a measure of ischemia 230 resulting from an occlusion that may be estimated in accordance with this example is an ischemic volume, i.e. a volume of (cerebral) tissue that is affected by an occlusion. This is a useful parameter in assessing the amount of damage that has occurred to the tissue as a result of an occlusion. An estimate of the ischemic volume may be obtained by calculating a volume of a sphere having a diameter that is equal to a length of the vessel over which the measurements of blood oxygen saturation 140 are below a threshold level. The length of the vessel in this example may correspond to the estimated length of the occlusion. Alternatively, the length of the vessel may be longer than the length of the occlusion in order to encompass the lack of blood flow in both proximal and distal positions with respect to the occlusion. Thus, different threshold values may be used to calculate the length of an occlusion, and to estimate of the volume of (cerebral) tissue that is affected by an occlusion.

In another related example, a temporal sequence of X-ray images is acquired contemporaneously with the optical data 120. In this example, the optical data 120 is generated by an intravascular optical probe 240 disposed in a blood vessel 170, and the one or more processors 110 are configured to:

receive X-ray image data, the X-ray image data comprising a temporal sequence of X-ray images representing the intravascular optical probe 240 in the blood vessel 170, and wherein the X-ray images are generated contemporaneously with the optical data 120;
identify, in the X-ray images, intravascular optical probe positions $210_{1..i}$ corresponding to the intravascular regions at which the measurements of blood oxygen saturation 140 are determined; and
wherein the outputting S130 the measurement of blood oxygen saturation 140 comprises outputting a graphical representation 250 of the blood vessel, and displaying in the graphical representation 250, an indication of the intravascular optical probe positions $210_{1..i}$ at which the corresponding measurements of blood oxygen saturation $140_{1..i}$ are determined.

By outputting the graphical representation 250 of the blood vessel, and displaying in the graphical representation 250 an indication of the intravascular optical probe positions $210_{1..i}$ at which the corresponding measurements of blood oxygen saturation $140_{1..i}$ are determined, the system provides a record of the blood oxygen saturation $140_{1..i}$ measurements. This information may be used by a physician to assess the length of the occlusion, or to assess the volume of (cerebral) tissue affected by the occlusion, and so forth. It also enables the physician to plan a subsequent treatment of the occlusion.

In this example, the X-ray images may be acquired using various types of X-ray imaging systems, such as for example a (spectral) projection X-ray imaging system, and a (spectral) computed tomography "CT" imaging system. The intravascular optical probe positions $210_{1..i}$ corresponding to the intravascular regions at which the measurements of blood oxygen saturation 140 are determined; may be identified using various tracking techniques. These include image processing-based tracking techniques such as feature detection to identify the position of the intravascular optical probe in the X-ray images. Machine learning techniques such as neural networks, may also be used. The intravascular optical probe may include a radiopaque marker to facilitate its identification in the X-ray images. Alternatively, a tracking system such as an electromagnetic tracking system, or an optical fiber-based tracking system, may be used to identify the intravascular optical probe positions $210_{1..i}$ in the X-ray images. The positions of the intravascular optical probe may be identified in the X-ray images based on tracking data provided by the tracking system, and based on a known a spatial relationship between a co-ordinate system of the tracking system and a co-ordinate system of the X-ray imaging system.

In this example, the graphical representation 250 of the blood vessel, may be provided in various forms, including for example in the form of an X-ray image, or in the form of a synthetic representation of the blood vessel.

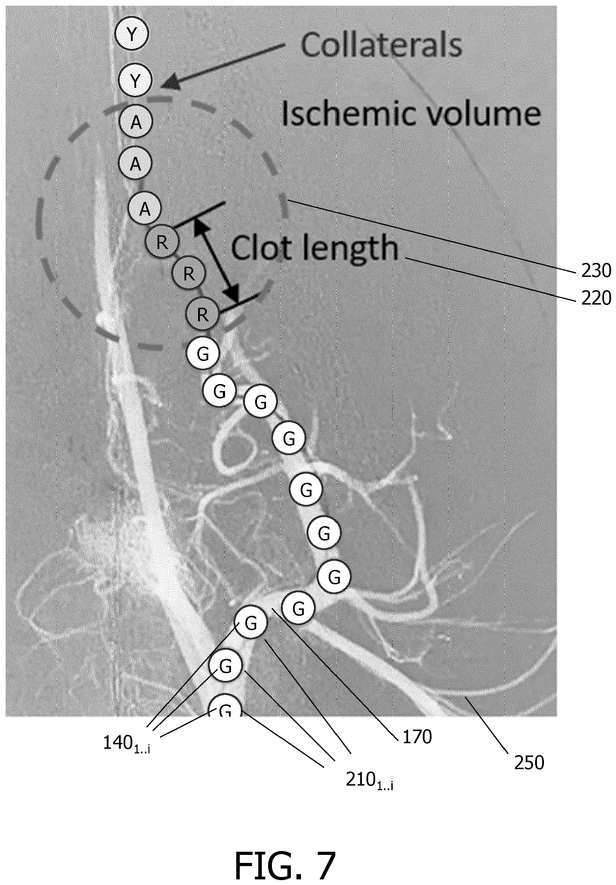

By way of an example, Fig. 7 is an example of a graphical representation 250 of a blood vessel 170 including an indication of intravascular optical probe positions $210_{1..i}$ and corresponding measurements of blood oxygen saturation, in accordance with some aspects of the present disclosure. In this example, the graphical representation 250 of the blood vessel is provided by a reference X-ray image. The reference X-ray image may be a current X-ray image, or a historic image, that is selected from the temporal sequence of X-ray images, for example. The intravascular optical probe positions $210_{1..i}$ are displayed in the reference X-ray image by mapping the positions from the X-ray images in the temporal sequence to the reference image based on a registration between each of the X-ray images in the temporal sequence and the reference X-ray image. In the example illustrated in Fig. 7, the measurements of blood oxygen saturation corresponding to the intravascular optical probe positions $210_{1..i}$ are displayed as circular icons, and the value of the blood oxygen saturation is indicated in the colors red (R), amber (A), and green (G). The measurements of blood oxygen saturation $140_{1..i}$ may alternatively be outputted in other ways in the graphical representation 250, such as in the form of a graph, or as a heatmap indicating the measurements and their corresponding positions along the blood vessel. As indicated in Fig. 7, other data may also be outputted in the graphical representation 250, such as a length 220 of an occlusion, and a measure of ischemia 230, for example.

Instead of outputting the graphical representation of the vessel in the form of an X-ray image as illustrated in Fig. 7, the graphical representation of the vessel may be provided by a synthetic representation of the blood vessel. For example, a (straight-line) schematic illustration of the blood vessel, may be provided, and the positions of the intravascular optical probe may be displayed in the synthetic representation of the blood vessel based on similar mapping and registration operations.

In another example, in addition to the measurement of blood oxygen saturation 140, the one or more processors 110 also determine a presence and/or a type of a tissue 190 in the intravascular region 130.

In this example, the one or more processors 110 are configured to:

analyze the optical data 120 to determine a presence and/or a type of a tissue 190 in the intravascular region 130; and
output an indication of the presence and/or the type of the tissue 190. The type of tissue that may be determined in accordance with this example may be a tissue such as a red blood cell-rich blood clot, a red blood cell-poor blood clot, a mixed clot, a vessel wall, and plaque, for example. As mentioned above, having information relating to the type of tissue present in the occlusion would enable the physician to make a first-time-right choice of the various treatment options and thereby perform early treatment.

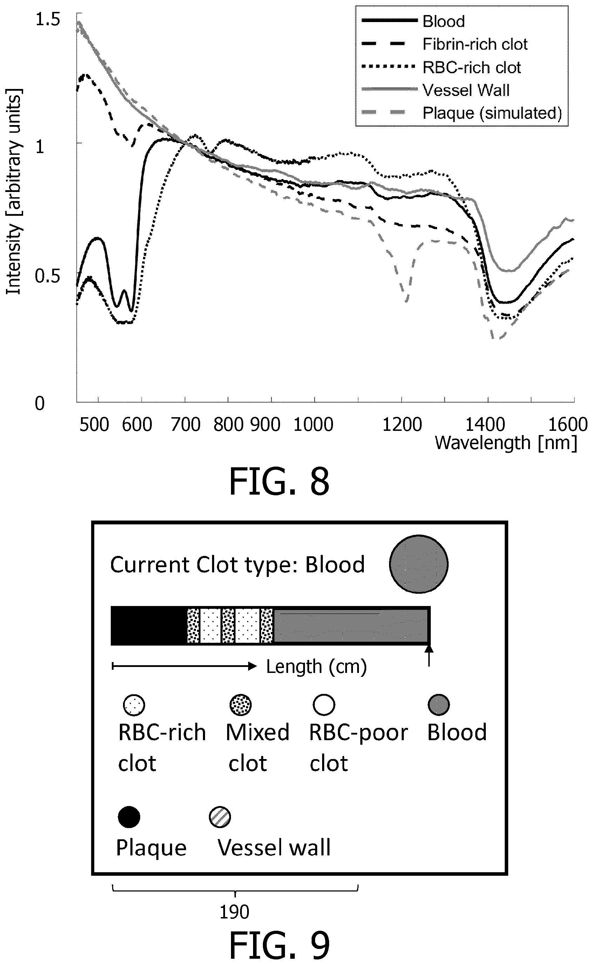

This example is described with reference to Fig. 8, which is an example of the optical signatures of various types of blood clot, blood, a vessel wall, and plaque, in accordance with some aspects of the present disclosure. The abscissa of the spectral signatures illustrated in Fig. 8 represents wavelength in nanometers, and the ordinate of the spectral signatures illustrated in Fig. 8 represents the reflectance of the various intravascular media. As may be appreciated from Fig. 8, differences between the spectra of a red blood cell-rich blood clot, a red blood cell-poor blood clot, vessel wall, blood, and plaque, may be used to distinguish these media from one another. A red blood cell-poor blood clot may alternatively be referred-to as a white clot, or a fibrin-rich blood clot. A red blood cell-rich blood clot may alternatively be referred to as a red clot. A mixture of a red blood cell-poor blood clot and a red blood cell-rich blood clot, may alternatively be referred-to as a mixed clot.

A distinction between a red blood cell-rich blood clot, a red blood cell-poor blood clot, and a mixed blood clot may be made from an optical spectrum of an intravascular region using the model fitting technique described with reference to Fig. 3. This model fitting technique may be used to determine values of the concentrations of the haemoglobin and lipid chromophores, i.e. $c_{Haemoglobin}$ and $c_{Lipid}$, and which represent the red blood cell content of the intravascular region, and the lipid content of the intravascular region, respectively. Thresholds may then be applied to the red blood cell content, and to the lipid content, in order to determine a presence and/or a type of a tissue 190 in the intravascular region 130. For instance, if the red blood cell fraction is below 20%, an intravascular region 130 may be deemed to be a red blood cell-poor blood clot. If the red blood cell fraction is above 30%, an intravascular region may be deemed to be a red blood cell-rich blood clot. If the red blood cell fraction is between 20% and 30%, an intravascular region may be deemed to be a mixed blood clot. The lipid content of an occlusion has also been found to be correlated with the presence of plaque. Thus, if the concentration of the lipid chromophores, i.e. $c_{Lipid}$, is above a specified threshold value, an intravascular region 130 may be deemed to be plaque.

The indication of the presence, or the type, of the tissue that is determined in this example may be outputted in various forms. For example, the type of the blood clot may be outputted in textual form, or in the form of an icon, or in the form of a graph. By way of an example, Fig. 9 is a schematic diagram illustrating an example of an outputted indication of various types of tissue 190, in accordance with some aspects of the present disclosure. In the upper portion of Fig. 9, the type of the tissue is outputted in textual form, and also as an icon. In the lower portion of Fig. 9, the type of the tissue is for optical data that has been obtained at a plurality of positions along a vessel. Outputting the tissue type for each of multiple positions along a vessel in accordance with this example facilitates an assessment of a length of an occlusion. This information is useful to a physician in determining a course of treatment for the occlusion. For instance it may be used to specify the length of a stent to deploy in the vessel after the occlusion has been removed.

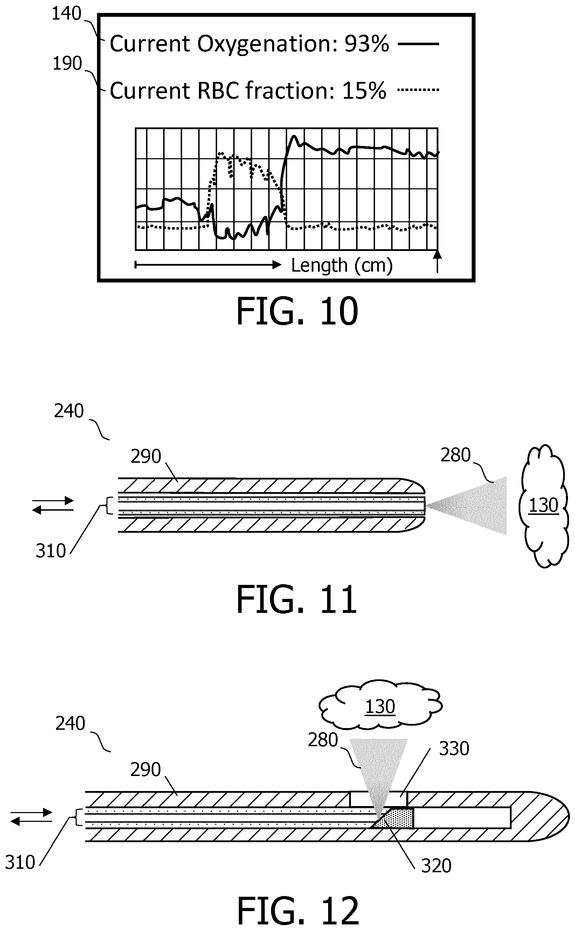

By way of another example, Fig. 10 is a schematic diagram illustrating an example of an outputted indication of a measurement of blood oxygen saturation 140 and an outputted indication of a red blood cell fraction, of an intravascular region, in accordance with some aspects of the present disclosure. In the example illustrated in Fig. 10, the measurements of blood oxygen saturation 140, and red blood cell fraction, are outputted for each of a plurality of positions along a vessel. The red blood cell fraction may be calculated from the concentration of haemoglobin, i.e. $c_{Haemoglobin}$, using the model fitting technique described above, and corresponds to the red blood cell content of the intravascular region. The red blood cell fraction serves as an indicator for the type of blood clot; relatively higher values of the red blood cell fraction representing red blood cell-rich blood clots, and relatively lower values of the red blood cell fraction representing red blood cell-poor blood clots.

The analysis of the optical data that is performed in this example in order to determine the tissue composition of the intravascular region presence and/or the type of the tissue 190, may therefore be performed in a similar manner to the analysis to determine the measurement of blood oxygen saturation140. Thus, a model fitting technique may be used, as described above with reference to Fig. 3. Alternatively, other optical models, other fitting techniques, a partial least squares "PLS" regression vector, or a machine learning algorithm such as a neural network, a decision tree, a support vector machine "SVM", principal component analysis, and so forth, may be trained, and subsequently used, to analyze the optical data.

In one example the one or more processors 110 are configured to determine the presence and/or the type of the tissue 190 by identifying a spectral signature of one or more of: a blood vessel wall, red blood cells, and plaque, in the optical spectrum. In this example, the operation of outputting an indication of the presence and/or the type of the tissue 190 comprises outputting an indication of the intravascular region 130 including one or more of: a blood vessel wall, a blood clot, and plaque, respectively.

In this example the optical data 120 that is analyzed may represent an optical spectrum of the intravascular region over one or more wavelength intervals that are within a range extending from approximately 450 nanometers to approximately 800 nanometers. As mentioned above, within this wavelength range, there are differences between the spectra that may be used to distinguish these media from one another. For example, these spectra have a characteristic absorption edge, and also characteristic absorption bands, that may be used to determine their individual contributions to a measured optical reflectance spectrum. Thus, within a range extending from approximately 450 nanometers to approximately 800 nanometers, i.e. over one or more wavelength intervals that are less than or equal to this range, these differences between the spectra may be used to distinguish between such media.

In a related example, if a result of the determining comprises outputting an indication of the intravascular region 130 including one or more of: a blood vessel wall, a blood clot, various actions may be taken in order to reduce the risk of an erroneous measurement of the blood oxygen saturation 140. In this example, if a result of the determining comprises outputting an indication of the intravascular region 130 including one or more of: a blood vessel wall, a blood clot, and plaque, the one or more processors 110 are configured to one or more of:

i) output an indication that the outputted measurement of blood oxygen saturation 140 is unreliable;
ii) suspend the outputting the measurement of blood oxygen saturation 140; and
iii) output a recommendation to obtain a revised measurement of the blood oxygen saturation 140 at a different intravascular region.

The detection of media within the intravascular region 130 such as a blood vessel wall, a blood clot, and plaque, indicates that the optical spectrum has been contaminated by the optical signatures of such media and may therefore be inaccurate. Consequently, such actions reduce the risk of an erroneous interpretation, or measurement, of the blood oxygen saturation 140.

As illustrated in Fig. 1, the optical data 120 that is used to determine the measurement of blood oxygen saturation 140, and likewise the presence and/or a type of a tissue 190 in the intravascular region 130, may be provided using at least one optical source 260, an intravascular optical probe 240 for insertion into a blood vessel, and at least one optical detector 270. These elements are now described with reference to Fig. 1 and Fig. 11 - Fig. 12.

In general, the at least one optical source 260 illustrated in Fig. 1 may generate optical radiation over a portion of the visible spectrum and/or over a portion of the near-infrared spectrum. In the example described above, the optical source(s) 260 may generate optical radiation over a first wavelength interval that is within a range extending from approximately 450 nanometers to approximately 800 nanometers, and/or over a second wavelength range that is within a range extending from approximately 1100 nanometers to approximately 1300 nanometers. In other examples, the optical source(s) may generate optical radiation over one or more wavelength intervals that are different to this example. In general, the optical source(s) may be provided by lamps, or light emitting diodes "LED"s, or lasers. The at least one optical detector 270 illustrated in Fig. 1 may be provided by various types of detectors, including for example a silicon detector, a gallium arsenide detector, an indium gallium arsenide detector, and so forth.

In some examples, the optical source(s) and the optical detector(s) are configured as a spectrometer. For instance, in one example, a spectrometer may be provided by using a diffractive or refractive optical element to spatially separate the optical radiation into different portions of the wavelength interval(s), and by using one or more optical detectors to detect the intensity the optical radiation over the different portions of the wavelength interval(s). The intensity of the collected optical radiation in each portion of the wavelength interval may be detected using multiple detectors, the spatial positions of which determine the detected portion of the wavelength interval(s), or alternatively a single detector may be used, the position of the single optical detector determining the detected portions of the wavelength interval(s).

In another example, a spectrometer may be provided by using a diffractive, or a refractive, optical element to select from the optical radiation generated by the optical source(s), optical radiation over the different portions of the wavelength interval(s). In this example, the spectrometer may be provided by temporally adjusting the position of the diffractive or refractive optical element so as to select optical radiation from the different portions of the wavelength interval(s) over time. A corresponding single optical detector may be used to temporally separate the detected optical radiation into the different portions of the wavelength interval(s).

In another example, a spectrometer may be provided by temporally modulating the intensity of different optical sources so as to selectively generate optical radiation over the different portions of the wavelength interval(s). A single optical detector may then be used to temporally separate the collected optical radiation into the different portions of the wavelength intervals.

In another example, a spectrometer may be provided by temporally switching filters that selectively transmit optical radiation over each of the different portions of the of the wavelength interval(s). The filters provide temporally modulated optical radiation for the different portions of the wavelength interval(s). A single optical detector may then be used to temporally separate the collected optical radiation into the different portions of the wavelength interval(s).

In one example, the at least one optical source and the at least one optical detector are configured as a Raman spectrometer. In another example, the at least one optical source and the at least one optical detector are configured to perform diffuse reflectance spectroscopy.

Returning to the system 100 illustrated in Fig. 1, the at least one optical source 260 is in optical communication with the intravascular optical probe 240, and the intravascular optical probe 240 is configured to irradiate the intravascular region 130 with optical radiation 280 generated by the at least one optical source 260, and to collect reflected optical radiation reflected by the intravascular region 130 in response to the irradiation. The at least one optical detector 270 is in optical communication with the intravascular optical probe 240, and the at least one optical detector 270 is configured measure an intensity of the collected optical radiation over the one or more wavelength intervals to provide the optical data 120.

Various types of intravascular optical probes may be used with the system illustrated in Fig. 1. In general, these include optical probes that are configured to measure one or more of: optical reflectance, optical scattering, optical transmission, and optical absorption. In general, the optical probe may include one or more optical fibers. For instance, in one example, the optical probe includes a single optical fiber 310, and the single optical fiber is used both to irradiate the intravascular region and to collect optical radiation returned by the intravascular region in response to the irradiation. In another example, the optical probe includes multiple optical fibers, and separate optical fibers are used to irradiate the intravascular region, and to collect optical radiation returned by the intravascular region in response to the irradiation. The use multiple optical fibers offers improved separation between the irradiating, and collected, optical radiation, at the expense of an increase in diameter, and reduced flexibility, of the optical probe. Some examples of optical probes that include a single optical fiber, and which are configured to measure optical reflectance, are described with reference to Fig. 11, and Fig. 12.

Fig. 11 is a schematic diagram illustrating a first example of an intravascular optical probe 240 for insertion into the vasculature, in accordance with some aspects of the present disclosure. The intravascular optical probe 240 illustrated in Fig. 11 includes an elongate body 290. The elongate body is flexible, and has a smooth outer surface in order to enable its insertion into the vasculature. The intravascular optical probe 240 illustrated in Fig. 11 includes a single optical fiber 310. The optical fiber is used both to irradiate an intravascular region 130 with optical radiation 280 and to collect optical radiation returned by the intravascular region in response to the irradiation. The optical fiber 310 is arranged within the elongate body 290. Optical radiation passing along the optical fiber 310 is emitted along a path that is parallel to the longitudinal axis of the optical probe. The optical fiber 310 also collects optical radiation that returns back along the same path as the emitted radiation. This enables the optical probe illustrated in Fig. 11 to analyze an intravascular region 130 that is disposed distally with respect to a distal end of the optical probe. The optical probe illustrated in Fig. 11 may therefore be referred-to as a "forward-sensing" optical probe.

By way of another example, Fig. 12 is a schematic diagram illustrating a second example of an intravascular optical probe 240 for insertion into the vasculature, in accordance with some aspects of the present disclosure. As in the Fig. 11 example, the intravascular optical probe 240 illustrated in Fig. 12 includes an elongate body 290. The elongate body is flexible, and has a smooth outer surface in order to enable its insertion into the vasculature. As in the Fig. 11 example, the intravascular optical probe 240 illustrated in Fig. 12 includes a single optical fiber 310. The optical fiber 310 is used both to irradiate an intravascular region 130 with optical radiation 280 and to collect optical radiation returned by the intravascular region in response to the irradiation. The optical fiber 310 is arranged within the elongate body 290. The optical fiber is arranged within the elongate body 290. In contrast to the Fig. 11 example, the intravascular optical probe 240 illustrated in Fig. 12 includes a reflective surface at a beveled end face 320 of the optical fiber 310. The reflective surface may be provided by a mirror, or total internal reflection, for example. The reflective surface redirects optical radiation passing along the optical fiber from a longitudinal direction with respect to the longitudinal axis of the optical probe, and into a radial direction with respect to the longitudinal axis of the optical probe. The emitted optical radiation 280 then passes through an aperture 330 in the elongate tube 290. The optical fiber 310 also collects optical radiation that returns back along the same path as the emitted radiation. This enables the optical probe illustrated in Fig. 12 to analyze an intravascular region 130 that is disposed radially with respect to a distal end of the optical probe. The optical probe illustrated in Fig. 12 may therefore be referred-to as a "radial-sensing" optical probe.

The optical probes illustrated in Fig. 11 and Fig. 12 may be used to analyze intravascular regions such as blood regions, and also occlusions in blood vessels, as illustrated in Fig. 6 above.

Instead of the arrangements illustrated in Fig. 11 and Fig. 12, other types of optical probes may be used with the system illustrated in Fig. 1. These alternative optical probes may have different numbers of optical fibers, and different arrangements of optical fibers. For instance, the distal ends of the optical fiber(s) may be oriented in a different direction to those illustrated in Fig. 11 and Fig. 12 in order to sense a different intravascular region. The distal end(s) of the optical fiber(s) may also be configured to measure another optical property of the intravascular region 130. For instance they may be configured to measure optical scattering, or optical transmission, or optical absorption.

Referring now to the system illustrated in Fig. 1, as mentioned above, the at least one optical source 260 is in optical communication with the intravascular optical probe 240, and the at least one optical detector 270 is also in optical communication with the intravascular optical probe 240. This optical communication may be provided by one or more optical components such as an optical connector, a beamsplitter, a bifurcated optical fiber, and so forth.

It is noted that the intravascular tissue analysis system 100 may, as illustrated in Fig. 1, also include one or more of: a display, such as the display 340 illustrated in Fig. 1, for displaying the measurement of blood oxygen saturation 140, the indication of the presence and/or the type of the tissue 190, and other outputs generated by the processor(s) 110; and a user input device (not illustrated in Fig. 1) configured to receive user input in relation to the operations performed by the one or more processors 110, such as a keyboard, a mouse, a touchscreen, and so forth.

In another example, a computer-implemented method of determining blood oxygen saturation, is provided. The method comprises:

receiving S 110 optical data 120 representing an optical spectrum of an intravascular region 130 over one or more wavelength intervals;
analyzing S 120 the optical data 120 to determine a measurement of blood oxygen saturation 140; and
outputting S 130 the measurement of blood oxygen saturation 140.

In another example, a computer program product is provided. The computer program product comprises instructions which when executed by one or more processors 110, cause the one or more processors to carry out a method of determining blood oxygen saturation. The method comprises:

receiving S 110 optical data 120 representing an optical spectrum of an intravascular region 130 over one or more wavelength intervals;
analyzing S 120 the optical data 120 to determine a measurement of blood oxygen saturation 140; and
outputting S 130 the measurement of blood oxygen saturation 140.

The above examples are to be understood as illustrative of the present disclosure, and not restrictive. Further examples are also contemplated. For instance, the examples described in relation to a system, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system (100) for determining blood oxygen saturation, the system comprising one or more processors (110) configured to:

receive (S110) optical data (120) representing an optical spectrum of an intravascular region (130) over one or more wavelength intervals;
analyze (S120) the optical data (120) to determine a measurement of blood oxygen saturation (140); and
output (S130) the measurement of blood oxygen saturation (140).

2. The system according to claim 1, wherein the one or more processors (110) are further configured to:

determine, based on the measurement of blood oxygen saturation (140), a perfusion status and/or a blood flow status of the intravascular region (130); and
output an indication of the perfusion status and/or the blood flow status, respectively.

3. The system according to claim 1 or claim 2, wherein the one or more processors (110) are further configured to:

classify, based on the measurement of blood oxygen saturation (140), the intravascular region (130) as representing a proximal position (150) with respect to an occlusion (160) in a blood vessel (170), or as a distal position (180) with respect to an occlusion (160) in a blood vessel (170); and
output an indication of the classification.

4. The system according to any one of claims 1 - 3, wherein the one or more processors (110) are further configured to:

analyze the optical data (120) to determine a presence and/or a type of a tissue (190) in the intravascular region (130); and

output an indication of the presence and/or the type of the tissue (190).

5. The system according to claim 4, wherein the one or more processors (110) are configured to determine the presence and/or the type of the tissue (190) by identifying a spectral signature of one or more of: a blood vessel wall, red blood cells, and plaque, in the optical spectrum; and

wherein the outputting an indication of the presence and/or the type of the tissue (190) comprises outputting an indication of the intravascular region (130) including one or more of: a blood vessel wall, a blood clot, and plaque, respectively.

6. The system according to claim 5, wherein if a result of the determining comprises outputting an indication of the intravascular region (130) including one or more of: a blood vessel wall, a blood clot, and plaque, the one or more processors (110) are further configured to one or more of:

   i) output an indication that the outputted measurement of blood oxygen saturation (140) is unreliable;
   ii) suspend the outputting the measurement of blood oxygen saturation (140); and
   iii) output a recommendation to obtain a revised measurement of the blood oxygen saturation (140) at a different intravascular region.

7. The system according to any previous claim, wherein the analyzing (S120) the optical data (120) comprises:

   fitting an optical model to the optical spectrum;
   extracting one or more model parameters from the fitted optical model; and
   determining the measurement of blood oxygen saturation (140) and/or the presence and/or the type of tissue (190) based on the one or more extracted model parameters.

8. The system according to any previous claim, wherein the optical data (120) represents an optical spectrum of the intravascular region (130) at each of a plurality of points in time; and

wherein the analyzing comprises:

   i) applying a temporal filter to at least a portion of the optical spectrum to provide one or more corresponding temporally-filtered spectra; and
   determining the measurement of blood oxygen saturation (140) using the temporally-filtered spectra;
   or
   ii) applying a temporal filter to the measurements of blood oxygen saturation (140), to provide temporally filtered measurements of blood oxygen saturation (140); and
   wherein the outputting the measurement of blood oxygen saturation (140) comprises outputting the temporally filtered measurements of blood oxygen saturation (140);
   or
   iii) extracting a pulsatile signal from temporal variations in the optical spectrum, and outputting the pulsatile signal;
   or
   iv) extracting a pulsatile signal from temporal variations in the measurement of blood oxygen saturation (140), and outputting the pulsatile signal.

9. The system according to any previous claim, wherein the optical data (120) represents an optical spectrum of the intravascular region at each of a plurality of positions ($210_{1..i}$) along a blood vessel; and

wherein the one or more processors (110) are configured to perform the analyzing, and the outputting, for a plurality of the positions along the blood vessel.

10. The system according to claim 9, wherein the plurality of positions comprises a proximal position (150) with respect to an occlusion (160) in the blood vessel (170), and a distal position (180) with respect to the occlusion (160) in the blood vessel (170).

11. The system according to claim 9 or claim 10, wherein the one or more processors (110) are further configured to:

   estimate, based on the measurements of blood oxygen saturation (140) at the plurality of positions along the blood vessel, a length (220) of an occlusion and/or a measure of ischemia (230) resulting from an occlusion; and

output the value of the length of the occlusion and/or the measure of ischemia.

12. The system according to any one of claims 9 - 11, wherein the optical data (120) is generated by an intravascular optical probe (240) disposed in a blood vessel (170), and wherein the one or more processors (110) are further configured to:

receive X-ray image data, the X-ray image data comprising a temporal sequence of X-ray images representing the intravascular optical probe (240) in the blood vessel (170), and wherein the X-ray images are generated contemporaneously with the optical data (120);

identify, in the X-ray images, intravascular optical probe positions ($210_{1..i}$) corresponding to the intravascular regions at which the measurements of blood oxygen saturation (140) are determined; and

wherein the outputting (S130) the measurement of blood oxygen saturation (140) comprises outputting a graphical representation (250) of the blood vessel, and displaying in the graphical representation (250), an indication of the intravascular optical probe positions (210i i) at which the corresponding measurements of blood oxygen saturation ($140_{1..i}$) are determined.

13. The system according to any previous claim, further comprising:

at least one optical source (260);

an intravascular optical probe (240) for insertion into a blood vessel; and

at least one optical detector (270);

wherein the at least one optical source (260) is in optical communication with the intravascular optical probe (240), and the intravascular optical probe (240) is configured to irradiate the intravascular region (130) with optical radiation (280) generated by the at least one optical source (260), and to collect reflected optical radiation reflected by the intravascular region (130) in response to the irradiation;

wherein the at least one optical detector (270) is in optical communication with the intravascular optical probe (240), and the at least one optical detector (270) is configured measure an intensity of the collected optical radiation over the one or more wavelength intervals to provide the optical data (120).

14. A computer-implemented method of determining blood oxygen saturation, the method comprising:

receiving (S 110) optical data (120) representing an optical spectrum of an intravascular region (130) over one or more wavelength intervals;

analyzing (S120) the optical data (120) to determine a measurement of blood oxygen saturation (140); and

outputting (S130) the measurement of blood oxygen saturation (140).

15. A computer program product comprising instructions which when executed by one or more processors (110), cause the one or more processors to carry out the method according to claim 14.

FIG. 1

S110

S120

S130

FIG. 2

FIG. 3

FIG. 4

FIG. 5

FIG. 6

FIG. 7

FIG. 8

FIG. 9

Current Oxygenation: 93% ——

Current RBC fraction: 15% ········

Length (cm)

**FIG. 10**

**FIG. 11**

130

**FIG. 12**

**EP 4 595 874 A1**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/067952 A2 (UNIV ILLINOIS [US]; GATTO RODOLFO [US] ET AL.) 14 June 2007 (2007-06-14) * paragraphs [0006] - [0008], [0015], [0018] - [0021], [0041], [0051], [0053], [0054]; claim 16; figure 3 * | 1-15 | INV. A61B5/00 A61B5/02 A61B5/1459 A61B5/024 A61B5/026 |
| A | US 2012/078117 A1 (OKADA KAZUNORI [JP] ET AL) 29 March 2012 (2012-03-29) * paragraph [0057] * | 1-15 | |
| A | MELISSA J SUTER ET AL: "Intravascular Optical Imaging Technology for Investigating the Coronary Artery", JACC: CARDIOVASCULAR IMAGING, ELSEVIER, AMSTERDAM, NL, vol. 4, no. 9, 14 March 2011 (2011-03-14), pages 1022-1039, XP028288763, ISSN: 1936-878X, DOI: 10.1016/J.JCMG.2011.03.020 [retrieved on 2011-07-19] * the whole document * | 1-15 | |
| A | US 9 814 393 B1 (MAO JIMMY JIAN-MIN [US] ET AL) 14 November 2017 (2017-11-14) * column 4, line 5 - line 29 * | 1-15 | TECHNICAL FIELDS SEARCHED (IPC) A61B |
| A | US 6 725 074 B1 (KAESTLE SIEGFRIED [DE]) 20 April 2004 (2004-04-20) * column 43, line 42 - line 52 * | 1-15 | |
| A | EP 3 482 684 A1 (KONINKLIJKE PHILIPS NV [NL]) 15 May 2019 (2019-05-15) * paragraphs [0004], [0006], [0008] * | 1-15 | |

-/--

The present search report has been drawn up for all claims

| Place of search | Date of completion of the search | Examiner |
|---|---|---|
| The Hague | 14 June 2024 | Papanikolaou, V |

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 15 4736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Wei Lu: "Wireless, implantable catheter-type oximeter designed for cardiac oxygen saturation", , 10 February 2021 (2021-02-10), XP093162521, Retrieved from the Internet: URL:https://www.science.org/doi/10.1126/sciadv.abe0579 [retrieved on 2024-05-14] * the whole document * ----- | 1-15 | |
| A | HAN-WOOK LEE ET AL: "The Periodic Moving Average Filter for Removing Motion Artifacts from PPG Signals 701 The Periodic Moving Average Filter for Removing Motion Artifacts from PPG Signals", INTERNATIONAL JOURNAL OF CONTROL, AUTOMATION, AND SYSTEMS, vol. 5, no. 6, 1 December 2007 (2007-12-01), pages 701-706, XP055256669, * the whole document * ----- | 1-15 | |
| X | US 2019/365248 A1 (MUELLER MANFRED [NL] ET AL) 5 December 2019 (2019-12-05) * paragraphs [0059], [0061], [0067], [0069], [0084]; figures 1, 4 * ----- | 1-15 | |
| X | US 2020/390375 A1 (ANDERSON ROBERT JOHN [US]) 17 December 2020 (2020-12-17) * paragraphs [0006], [0071] - [0074] * ----- | 1-15 | **TECHNICAL FIELDS SEARCHED (IPC)** |

The present search report has been drawn up for all claims

| Place of search | Date of completion of the search | Examiner |
|---|---|---|
| The Hague | 14 June 2024 | Papanikolaou, V |

EPO FORM 1503 03.82 (P04C01)

## ANNEX TO THE EUROPEAN SEARCH REPORT
## ON EUROPEAN PATENT APPLICATION NO.

EP 24 15 4736

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2024

| Patent document cited in search report | | | Publication date | Patent family member(s) | | | Publication date |
|---|---|---|---|---|---|---|---|
| WO 2007067952 | A2 | | 14-06-2007 | US | 2008300493 | A1 | 04-12-2008 |
| | | | | WO | 2007067952 | A2 | 14-06-2007 |
| US 2012078117 | A1 | | 29-03-2012 | CN | 102421376 | A | 18-04-2012 |
| | | | | EP | 2430981 | A1 | 21-03-2012 |
| | | | | JP | 5658146 | B2 | 21-01-2015 |
| | | | | JP | WO2010131713 | A1 | 08-11-2012 |
| | | | | RU | 2011150518 | A | 20-06-2013 |
| | | | | US | 2012078117 | A1 | 29-03-2012 |
| | | | | WO | 2010131713 | A1 | 18-11-2010 |
| US 9814393 | B1 | | 14-11-2017 | US | 8792951 | B1 | 29-07-2014 |
| | | | | US | 9814393 | B1 | 14-11-2017 |
| | | | | US | 10905332 | B1 | 02-02-2021 |
| | | | | US | 11484209 | B1 | 01-11-2022 |
| US 6725074 | B1 | | 20-04-2004 | EP | 1196862 | A1 | 17-04-2002 |
| | | | | JP | 4495378 | B2 | 07-07-2010 |
| | | | | JP | 2003505120 | A | 12-02-2003 |
| | | | | US | 6725074 | B1 | 20-04-2004 |
| | | | | WO | 0077659 | A1 | 21-12-2000 |
| EP 3482684 | A1 | | 15-05-2019 | EP | 3482684 | A1 | 15-05-2019 |
| | | | | EP | 3706628 | A1 | 16-09-2020 |
| | | | | WO | 2019091816 | A1 | 16-05-2019 |
| US 2019365248 | A1 | | 05-12-2019 | EP | 3573514 | A1 | 04-12-2019 |
| | | | | JP | 7426823 | B2 | 02-02-2024 |
| | | | | JP | 2020505979 | A | 27-02-2020 |
| | | | | US | 2019365248 | A1 | 05-12-2019 |
| | | | | US | 2023148874 | A1 | 18-05-2023 |
| | | | | WO | 2018137949 | A1 | 02-08-2018 |
| US 2020390375 | A1 | | 17-12-2020 | US | 2017196486 | A1 | 13-07-2017 |
| | | | | US | 2020390375 | A1 | 17-12-2020 |
| | | | | WO | 2017123764 | A1 | 20-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FARREL, T. J. et al.** A diffusion theory model of spatially resolved, steady-state diffuse reflectance for the noninvasive determination of tissue optical properties in vivo. *Medical Physics*, 1992, vol. 19 (4), 879-888

- **VERKRUYSSE, W. et al.** Modelling light distributions of homogeneous versus discrete absorbers in light irradiated turbid media. *Phys. Med. Biol*, vol. 42, 51